# EUROPEAN PATENT SPECIFICATION

(11) **EP 1 458 364 B1**
(45) Date of publication and mention of the grant of the patent: **27.02.2008**
(21) Application number: 02791739.2
(22) Date of filing: 29.11.2002
(51) Int. Cl.: A61K 9/20, A61K 31/64

(54) **PHARMACEUTICAL COMPOSITIONS COMPRISING METFORMINE AND GLIBENCLAMIDE FOR THE TREATMENT OF TYPE-II DIABETES MELLITUS**
PHARMAZEUTISCHE ZUSAMMENSETZUNGEN MIT METFORMIN UND GLIBENCLAMID ZUR BEHANDLUNG VON DIABETES MELLITUS TYP II
COMPOSITIONS PHARMACEUTIQUES COMPRENANT LE GLIBENCLAMIDE ET LA METFORMINE DESTINEES AU TRAITEMENT DU DIABETE SUCRE DE TYPE II

(30) Priority: 29.11.2001 IT FI20010023
(43) Date of publication of application: 22.09.2004
(73) Proprietor: Menarini International Operations Luxembourg S.A., 1611 Luxembourg (LU)
(72) Inventor: TOSETTI, Alessandro, I-50012 Bagno A Ripoli (IT); GUIDUCCI, Mauro, I-52100 Arezzo (IT); VITI, Giovanni, I-50127 Firenze (IT)
(74) Representative: Gervasi, Gemma
(86) International application number: PCT/EP2002/013497
(87) International publication number: WO 2003/045355

(56) References cited:
- WO-A-00/03742
- WO-A-01/82875
- WO-A-97/17975
- WO-A-02/055009
- FR-A- 2 244 481

## Description

### Field of the invention

The present invention relates to new pharmaceutical compositions comprising glibenclamide and metformin, effective for the treatment of type-II diabetes mellitus, wherein the two active ingredients are maintained separate from one another although within the same composition.

### State of the art

Non-insulin dependent type-II diabetes is a metabolic disorder characterised by hyperglycaemia, linked to an insulin deficiency, a resistance of peripheral tissues to insulin itself, an increase in the production of glucose by the liver, a reduced glucose tolerance.

Two major categories of antidiabetic drugs exist: the sulphonylureas, amongst which we note glibenclamide, chlorpropamide, glicazide, tolazemide, gliquidone, glipizide, tolbutamide and the biguanides, among which we note fenformin and metformin. Whilst the sulphonylureas act through the stimulation of the production of insulin by the pancreas, and a residual activity of this organ is necessary for this category of drugs to be effective, the biguanides act through the increase of the use of glucose, reducing the insulin-resistance and hepatic glucogenesis.

Therefore for this category of drug to have any effect, a residual insulin production by the beta cells of the pancreas is necessary.

It is known that the initial treatment of type-II diabetes can be uniquely based on the control of diet and physical exercise, but frequently these lifestyle changes are insufficient for an optimal control of glycaemia such that the use of said oral antidiabetic drugs become necessary.

The treatment of type-II diabetes mellitus by monotherapy with an oral antidiabetic drug can be effective in the long term but, for a broad percentage of patients the effectiveness is progressively reduced over the years.

Having been always more frequently demonstrated in type-II diabetes patients there is an agreement between the insulin production deficiency and the peripheral insulin resistance, so that the sulphonylureas and biguanides possess complimentary modes of action, it is evident that the treatment consisting of the association of the two said categories of drugs are today already consolidated. Furthermore, to improve the compliance of the patient, tablets containing the two drugs already in association have been produced.

The combined sulphonylureas and biguanides treatment assumes therefore an important role in the therapy of type-II diabetes, in that it allows an improved metabolic control in these patients in which biguanides or sulphonylureas alone have shown themselves ineffective with passing time.

Amongst the two categories of said drugs, the biguanides and sulphonylureas, those most used for the treatment of type-II diabetes are metformin and glibenclamide respectively.

The association of metformin and glibenclamide presents notable advantages with respect to the administration of the two active ingredients separately, since it significantly improves the 'compliance' and in addition, especially in older patients and/or in patients subjected to polytherapy with other drugs, it reduces the risk of errors in administration.

Today, five combinations which envisage the presence of a single tablet of glibenclamide and metformin in different dosages are present on the market, in particular:
1) glibenclamide at a dosage of 1.25 mg and metformin hydrochloride at a dosage of 250 mg (glibenclamide/metformin weight ratio of 1:200);
2) glibenclamide at a dosage of 2.5 mg and metformin hydrochloride at a dosage of 500 mg (glibenclamide/metformin weight ratio of 1:200);
3) glibenclamide at a dosage of 5 mg and metformin hydrochloride at a dosage of 1000 mg (glibenclamide/metformin weight ratio of 1:200);
4) glibenclamide at a dosage of 2.5 mg and metformin hydrochloride at a dosage of 400 mg (glibenclamide/metformin weight ratio of 1:160);
5) glibenclamide at a dosage of 5 mg and metformin hydrochloride at a dosage of 500 mg (glibenclamide/metformin weight ratio of 1:100).

From the most recent data in the literature it emerges that the maximum recommended daily dosage of glibenclamide for the most serious and poorly controlled patients is 20 mg, in one or more administration. Such a dosage, in order to obtain an optimal therapeutic effect, can be combined with a daily dosage of metformin comprised of between 1000 and 2000 mg. The metformin is excreted unmodified in the urine; it is in fact not subjected to hepatic metabolisation (in fact no metabolites are found in humans) nor to biliary excretion. In subjects with reduced renal function (based on creatinine clearance) the plasma half-life of metformin is prolonged and the renal clearance is diminished in proportion to the reduction of the clearance of creatinine.

In view of the different optimal dosages, it is evident that the ratio between metformin and glibenclamide in association with each other must be maintained in a ratio of around 100:1 or greater, we are therefore in general in the presence of a massive dosage of metformin associated with relatively modest dosages of glibenclamide.

Also the solubility in water is notably different between the two active ingredients and, whilst metformin is extremely soluble in water, glibenclamide is relatively poorly soluble.

The simplest way of association between the two active ingredients is that of mixing them closely together with appropriate excipients.

Vigneri et al. (Diabete & Metabolisme, 1991, 17, 232-234) have proposed an association of the two compounds in a daily dosage of 1500 mg of metformin and 15 mg of glibenclamide.

In WO9717975 the use of an association of the two ingredients in a ratio of 100:1 is claimed for the treatment of type-II diabetes mellitus in the case of failure due combinations having a higher ratio in metformin.

In WO0003742, the inventors of LIPHA SA have clearly demonstrated that simple mixing of metformin and glibenclamide in ratios of 100:1 with certain excipients, resulted in a heavy reduction in the bioavailability of the glibenclamide, visible through the area under the curve (AUC) with respect to that obtainable with a co-administration of the same active ingredients in the same dosages. The inventors have succeeded in avoiding this problem by micronising the glibenclamide prior to adding it to the mixture.

The technique of micronising is also followed by Bristol-Myers Squibb for the preparation of commercial associations with the trade name Glucovance^{®} in which, using particles of glibenclamide with average dimensions still more reduced than those described in WO0003742, they manage to achieve an AUC which is even superior, even if by little, to that of non-micronised glibenclamide, sold under the trade name Micronase, when given in co-administration with metformin (Summary of Product Characteristics of Glucovance^{®} distributed by Bristol-Myers Squibb through their Internet site, as sales support, starting from 31/07/2000).

Since that the advantages of the administration in association of metformin and glibenclamide, or salts thereof, are now evident, the need to resolve the problem of reduced bioavailability of glibenclamide when this is mixed with metformin is very much felt.

### Summary of the invention

The Applicant has now surprisingly found that preparing pharmaceutical compositions comprising metformin and glibenclamide, or salts thereof, as active ingredients so as that the two active ingredients are maintained separated from each other, there is not, as the consequence of the association of these active ingredients with high metformin/glibenclamide ratios, a heavy reduction in the bioavailability of the glibenclamide.

An effective way to maintain the two active ingredients separate has been found by the Applicant in the preparation of tablets, for example multi-layered tablets (two or more layers, in each of which is exclusively one of the two drugs) or metformin tablets uniformly coated with glibenclamide.

The advantageous characteristics of the formulations in the invention have in addition been further improved by the selection of appropriate pharmaceutically acceptable excipients.

Therefore the subject of the present invention are orally administrable pharmaceutical formulations in the form of tablets, comprising as active ingredients glibenclamide and metformin, or pharmaceutically acceptable salts thereof, characterised by the fact that said active ingredients are comprised in layers separated from each other.

The characteristics and the advantages of the pharmaceutical compositions according to the present invention will be illustrated in the detailed description that follows.

### Detailed description of the invention

The simplest way for the preparation of an association of two drugs for which a different location or a different method of absorption is not envisaged is surely that of mixing them closely together, but in the case of glibenclamide with metformin, as said above, the absorption of glibenclamide is unexplainably hindered by the massive presence of metformin.

The evaluation of the absorption is always quite complex, but, in the first approximation, we can consider the percentage of dissociation of the tablet containing the active ingredient as an indication of the absorption that we might expect (J. Pharm. Pharmacol. 2000, 52, 831-838, Drug Development and Industrial Pharmacy, 1993, 19 (20), 2713-2741).

Micronisation of the glibenclamide, as one in the art might deduce from the solution proposed in the International Patent Application No. WO0003742 and from the solution described by Bristol Myers Squibb, does not seem to be generally satisfactory; in our case the micronisation of the glibenclamide according to the specifics adopted by us, even if different from the conditions used in the two methods mentioned above, has not been sufficient to make the tablets containing a mixture of glibenclamide and metformin dissolve in a comparable way to that of the tablets comprising glibenclamide and metformin separated from each other.

By maintaining the two active ingredients separate from each other, but in the same pharmaceutical formulation, it has been surprisingly possible to resolve the problem allowing the dissolution of the glibenclamide in a comparable or even higher way to that which would have been obtained by administering the two active ingredients in two completely separate formulations. The maintenance of the two active ingredients in a single formulation guarantees greater 'compliance' with the patient and at the same time can guarantee a therapeutic treatment comparable to that with the two active ingredients separated, without the risk of under dosing for glibenclamide (in the change from a therapy with two separate drugs to that with the two drugs mixed together).

To obtain formulations in which the two active ingredients are maintained separated from each other is technically a little more complex than simply mixing them, but the advantages that have surprisingly been found render the formulations of the invention of great interest.

Not all the formulations in which the two active ingredients are maintained separated constitute equally satisfactory solutions for the present invention. It would in fact be auspicious that the part containing glibenclamide dissolves quickly in the medium. One way to reach this speed of dissolution, is that of having a large erosion surface and to maintain this surface as large as possible with time until the complete dissolution is achieved.

There are two preferred pharmaceutical forms according to the present invention:
1) multilayered tablets, preferably selected from:
   a) bi-layered tablets in which the glibenclamide is compressed into a relatively thin layer over a tablet of metformin, and
   b) tri-layered tablets in which the glibenclamide is compressed into two layers positioned over and under a central layer of metformin,
2) coated tablets, preferably selected from:
   a') tablets of metformin uniformly coated by a thin layer containing glibenclamide which is compressed all around it, and
   b') tablets of metformin externally painted with a solution of glibenclamide which layers over it.

In the embodiment of the present pharmaceutical formulations glibenclamide in micronised form or otherwise, can be used indifferently.

We can better understand what above said by observing the exemplifying schematic diagram below. 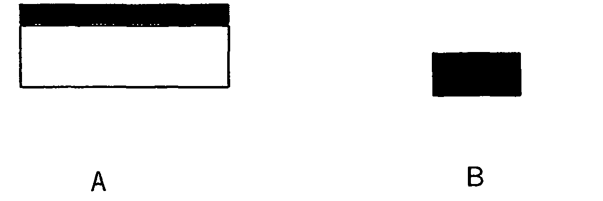 A and B represent transverse sections of two cylindrical tablets: A represents a bilayer in which the dark part is the layer containing glibenclamide, and B represents a tablet of just glibenclamide, but with the same total volume of the part containing glibenclamide as A.

The tablets schematised as B, currently on the market or commonly realisable by any person skilled in the art, containing from 1.25 to 5 mg of glibenclamide (for a total weight, including excipients, of 100 - 150 mg with a maximum of 200 mg) present a surface exposed to erosion less than, or at the most comparable to, the surface offered by the layer of glibenclamide compressed over the support of metformin, as outlined in A. But what is more important is that the erosion surface of B reduces rapidly during dissolution, whilst in case A the reduction of the surface is negligible at the beginning of the process and is discernible only in the advanced stages of dissolution. As a consequence the dissolution kinetics of the glibenclamide in A will be much faster than in B and the differences in dissolution between the two tablets will increase with time, as the difference between the erodible surfaces of A and of B increases.

Also the other solutions described in points 1 and 2 show kinetic advantages analogous to these described for the bi-layered tablet in figure A.

For the above stated reasons it is particularly efficacious for the purposes of the invention, and it is therefore to be considered as a particularly preferred solution of the invention, the multi-layered tablet, more preferably the bi-layered or tri-layered tablet of type 1.

As previously mentioned, the bioavailability of the glibenclamide in association with metformin has a complex behaviour which can be also influenced by other factors in addition of the mixing with metformin: in fact, it is a further subject of the present invention also the selection of appropriate excipients and/or additives which serve to increase the dissolution of the portion containing glibenclamide and consequently its bioavailability.

Furthermore, the fundamental choice of keeping the two active ingredients separated, allows to dosage the excipients and/or the additives necessary to obtain the desired bioavailability of the glibenclamide, only into the layer comprising the glibenclamide, thereby reducing their use to that which is strictly necessary, and avoiding potential problems with the metformin.

The tablets realisable according to the present invention are suitable for amounts of glibenclamide ranging between 1 and 20 mg for each tablet, preferably between 2.5 and 5 mg; and for amounts of metformin ranging between 200 and 1000 mg for each tablet, and preferably between 500 and 850 mg.

The present formulations in tablet form can be prepared according to the methodologies commonly used in pharmaceutical techniques. The bi-layered tablets of the invention, for example, can be prepared in the following manner: the compositions of the layer containing metformin and of the layer containing glibenclamide are prepared separately; the first can be obtained by mixing metformin powders previously sieved and appropriate pharmaceutically acceptable excipients, then granulating such a mixture together with one or more dispersing agents, drying the so obtained granulate, which is then calibrated and added with one or more lubricating agents. The composition of the glibenclamide layer can be instead prepared by directly mixing such active ingredient with appropriate pharmaceutically acceptable excipients and then sieving the mixed powders. Then using a press suitable for the production of bi-layered tablets, the compression of the above said compositions is achieved.

The coated tablets of the invention can be realised preparing a metformin nucleus by compression of the above said composition, then uniformly coating this nucleus with a thin layer containing glibenclamide by compression of the above said composition containing glibenclamide or by painting the nucleus with a solution of glibenclamide.

Preferred excipients for the portion containing glibenclamide in tablet form according to the invention are surfactants and hydrosoluble dispersing agents.

In the case of surfactants, an amount ranging between 0.1 and 10 mg can be used in the part of each tablet containing glibenclamide, preferably between 0.5 and 2 mg; the preferred surfactants according to the invention are selected from the alkaline or alkaline earth salts of lauryl sulphate and of Polysorbate 80. A particularly preferred surfactant is sodium lauryl sulphate.

In the case of the hydrosoluble dispersing agents, these can be used in quantities comprised of between 20 and 100 mg in the part of each tablet containing glibenclamide, preferably between 45 and 55 mg; the preferred hydrosoluble dispersing agents are selected from the group of the polyethylene glycols, among these particularly preferred is Macrogol 6000.

When a solid surfactant is used, such as a salt of lauryl sulphate, the moist surfactant can be possibly dispersed in the lubricating agent (for example Mg stearate) prior to using it in the granulation, in order to improve the wetting effect of the surfactant itself.

When a hydrosoluble dispersing agent is used, it can optionally be preferable to melt the dispersing agent (for example Macrogol 6000), then disperse the glibenclamide in it, allow to solidify again, pulverise the so obtained solid, and use it for the later preparation of the granulate.

The following examples are reported for the purposes of illustration of the present invention.

### EXAMPLE 1 (COMPARISON)

| **Preparation of 5 mg Glibenclamide tablets** | |
|---|---|
| Composition of each tablet in mg: | |
| Glibenclamide " | 5 |
| Pre-gelatinised corn starch | 58.3 |
| Hydrated colloidal silica | 0.5 |
| Lactose monohydrate | 96.2 |
| Na lauryl sulphate | 1 |
| Talc " | 0.5 |
| Magnesium stearate " | 0.5 |

### Preparation:

The mixture of the active ingredients and excipients is subjected to granulation and the resulting granulate is dried, then subjected to compression until obtaining tablets each containing 5 mg of glibenclamide and having the above reported composition.

### EXAMPLE 2 (COMPARISON)

| **Preparation of 400 mg Metformin tablets** | |
|---|---|
| Composition of each tablet in mg: | |
| Metformin HCl | 400 |
| Microcrystalline cellulose | 65 |
| Corn starch | 60 |
| Anhydrous colloidal silica | 20 |
| Gelatine | 40 |
| Glycerine | 17.5 |
| Talc | 17.5 |
| Magnesium stearate | 7.5 |
| Cellulose-acetate phthalate | 2 |

The mixture of the active ingredients and the excipients is subjected to granulation and the resulting granulate is dried, then subjected to compression so as to obtain tablets each containing 400 mg of metformin and having the above reported composition.

### EXAMPLE 3 (COMPARISON)

| Preparation of tablets containing the mixture of Glibenclamide + Metformin (5 + 400 mg) | |
|---|---|
| Composition of each tablet in mg: | |
| Metformin HCl " | 400 |
| Glibenclamide | 5 |
| Microcrystalline cellulose | 65 |
| Corn starch | 55 |
| Pre-gelatinised corn starch | 58.3 |
| Anhydrous colloidal silica | 20 |
| Hydrated colloidal silica | 0.5 |
| Lactose monohydrate | 96.2 |
| Gelatine | 40 |
| Glycerine | 17.5 |
| Talc | 18 |
| Magnesium stearate | 8 |
| Cellulose acetate phthalate | 2 |
| Na lauryl sulphate | 1 |
| Diethylphthalate | 0.5 |

The two active ingredients and the excipients have been granulated together. The granulate thus obtained is dried and, upon completion of drying, is calibrated. To the calibrated granulate is added the Mg stearate and is mixed. The mixture obtained is subjected to fine granulation so as to obtain tablets, each having the above reported compositions.

### EXAMPLE 4 (COMPARISON)

| Preparation of separated tablets of 5 mg of Glibenclamide and 400 mg of Metformin: | |
|---|---|
| Metformin tablet | |
| Metformin HCl 500 | |
| Macrogol 4000 | 24.7 |
| Anhyd. coll. silica-Aerosil^{®} 200- | 5 |
| Polyvinylpyrrolidone K 25 | 20 |
| Mg stearate | 5.9 |
| Glibenclamide tablet | |
| Glibenclamide | 5 |
| Lactose mon.-granul. | 128 |
| Corn starch | 40 |
| Microgr. Cell.-Avicel^{®} PH 102- | 15 |
| HPMC -5- | 4.8 |
| Na croscarmellose 13 | |
| Anhydr. coll. silica-Aerosil^{®} 200- | 0.7 |
| Polysorbate 80 | 0.5 |
| Mg stearate | 1 |
| Yellow iron oxide | 1 |
| Red iron oxide | 1 |

The separated tablets have been prepared as described above in Examples 1 and 2.

### EXAMPLE 5 (COMPARISON)

| Preparation of tablets containing the mixture Glibenclamide + Metformin (5 + 400 mg) (separate granulation) | |
|---|---|
| Composition of each tablet in mg: | |
| Metformin HCl | 500 |
| Macrogol 4000 | 24.7 |
| Anhyd. coil. silica -Aerosil^{®} | 200-5 |
| Polyvinylpyrrolidone K 25 | 20 |
| Mg stearate | 5.9 |
| Glibenclamide | 5 |
| Lactose mon.-granul. | 200-128 |
| Corn starch | 40 |
| HPMC-5- | 4.8 |
| Na croscarmellose | 13 |
| Anhyd. coll. silica -Aerosil^{®} | 200-0.7 |
| Polysorbate 80 | 0.5 |
| Mg stearate | 1 |
| Yellow iron oxide | 1 |
| Red iron oxide | 1 |

The two active ingredients have been granulated separately, each with their respective excipients (analogous to Example 4), but then have been mixed together and subjected to compression to form tablets each having the above reported composition.

### EXAMPLE 6 (COMPARISON)

| Preparation of tablets containing the mixture Glibenclamide + Metformin (5 + 500 mg) | |
|---|---|
| Composition of each tablet in mg: | |
| Metformin HCl | 500 |
| Macrogol 4000 | 24.7 |
| Polyvinylpyrrolidone K 25 | 20 |
| Glibenclamide | 5 |
| Lactose mon.-granul. | 40 |
| Microgr. Cell.-Avicel^{®} PH | 102-15 |
| HPMC -5- | 4.8 |
| Na croscarmellose | 13 |
| Anhyd. coll. silica -Aerosil^{®} | 200- 5.7 |
| Polysorbate 80 | 0.5 |
| Mg stearate | 6.9 |
| Yellow iron oxide | 1 |
| Red iron oxide | 1 |

The preparation is analogous to that reported above for Example 3.

### EXAMPLE 7

| Preparation of Glibenclamide + Metformin (5 + 500 mg) bi-layered tablets | |
|---|---|
| Composition of the Metformin layer for each tablet, in mg: | |
| Metformin HCl | 500 |
| Macrogol 4000 | 24.7 |
| Polyvinylpyrrolidone | 20 |
| Anhydrous colloidal silica | 5 |

| Composition of the Glibenclamide layer for each tablet, in mg: | |
|---|---|
| Glibenclamide | 5 |
| Lactose monohydrate | 50.75 |
| Na lauryl sulphate | 0.75 |
| Microcrystalline cellulose | 100 |
| Sodium croscarmellose | 5.5 |
| Anhydrous colloidal silica | 0.5 |
| Magnesium Stearate | 3.4 |
| Yellow iron oxide colouring E 172 | 1 |
| Red iron oxide colouring E 172 | 1 |

### Preparation:

### Metformin layer

- Granulating solution: dissolve the Macrogol 4000 under stirring.
- Granulation: sieve the metformin, Polyvinylpyrrolidone and anhydrous colloidal silica.
- Mix the sieved powders, then granulate using the previously prepared granulating solution. Dry the so obtained granulate and, once the drying procedure is completed, calibrate the same.
- Final mixture: add the Mg stearate to the calibrated granulate and mix.

### Glibenclamide layer

- Mixture preparation: mix the anhydrous colloidal silica, glibenclamide, Na lauryl sulphate, Na croscarmellose, yellow iron oxide colouring, red iron oxide colouring and c.a. 20% of lactose monohydrate. Sieve the mixed powders, Microcrystalline Cellulose and the remaining lactose monohydrate. Mix, add the Mg stearate and mix again.

### Bi-layered tablet

Using a press suitable for the production of bi-layered tablets, the two layers are compressed.

The tablets thus realised have the following values in disgregation test:
glibenclamide layer: complete disgregation in less than one minute (30 seconds on average)
metformin layer: complete disgregation in 10 minutes on average.

### EXAMPLE 8

| Preparation of Glibenclamide + Metformin (5 + 500 mg) bi-layered tablets | |
|---|---|
| Composition of the Metformin layer for each tablet, in mg: | |
| Metformin HCl | 500 |
| Macrogol 4000 | 24.7 |
| Anhyd. coll. Silica-Aerosil^{®} | 200-5 |
| Polyvinylpyrrolidone K 25 | 20 |
| Mg stearate | 5.9 |

| Composition of the Glibenclamide layer for each tablet, in mg: | |
|---|---|
| Glibenclamide | 5 |
| Macrogol 6000 | 50 |
| Lactose mon. S.D. | 34 |
| Microgr. Cell.-Avicel® PH | 102-70 |
| Na croscarmellose | 5 |
| Coll. silica anhydr.-Aerosil^{®} | 200-0.5 |
| Mg stearate | 0.5 |

The preparation is analogous to that reported above for Example 7.

### EXAMPLE 9

| Preparation of Glibenclamide + Metformin (5 + 500 mg) bilayered tablets | |
|---|---|
| Composition of the Metformin layer for each tablet, in mg: | |
| Metformin HCl | 500 |
| Macrogol 4000 | 24.7 |
| Coil. silica anhyd.-Aerosil^{®} | 200- 5 |
| Polyvinylpyrrolidone K 25 | 20 |
| Mg stearate | 5.9 |

| Composition of the Glibenclamide layer for each tablet, in mg: | |
|---|---|
| Glibenclamide | 5 |
| Micr. Cell.-Avicel^{®} PH | 102-100 |
| Na croscarmellose | 5.25 |
| Anhydr. coll. silica -Aerosil^{®} | 200-0.5 |
| Na lauryl sulphate | 0.5 |
| Mg stearate | 0.5 |
| Yellow iron oxide | 1 |
| Red iron oxide | 0.75 |

The preparation is analogous to that reported above for Example 7.

### EXAMPLE 10

| Preparation of Glibenclamide + Metformin (5 + 400 mg) bilayered tablets | |
|---|---|
| Composition of the Metformin layer for each tablet, in mg: | |
| Metformin HCl | 400 |
| Microcrystalline cellulose | 65 |
| Corn starch | 60 |
| Anhydrous colloidal silica | 20 |
| Gelatine | 40 |
| Glycerine | 17.5 |
| Talc | 17.5 |
| Magnesium stearate | 7.5 |
| Cellulose acetate phthalate | "2 |
| Diethylphthalate | 0.5 |

| Composition of the Glibenclamide layer for each tablet, in mg: | |
|---|---|
| Glibenclamide " | 5 |
| Pre-gelatinised corn starch | 58.3 |
| Hydrated colloidal silica | 0.5 |
| Lactose monohydrate | 96.2 |
| Na lauryl sulphate | 1 |
| Talc " | 0.5 |
| Magnesium stearate " | 0.5 |

The preparation is analogous to that reported above for Example 7.

### EXAMPLE 11

| Preparation of Glibenclamide + Metformin (5 + 500 mg) bi-layered tablets | |
|---|---|
| Composition of the Metformin layer for each tablet, in mg: | |
| Metformin HCl | 500 |
| Macrogol 4000 | 24.7 |
| Anhydr. coll. silica -Aerosil^{®} | 200-5 |
| Polyvinylpyrrolidone K 25 | 20 |
| Mg stearate | 5.9 |

| Composition of the Glibenclamide layer for each tablet, in mg: | |
|---|---|
| Glibenclamide | 5 |
| Lactose mon.-granul. | 128 |
| Corn starch | 40 |
| Microgr. Cell.-Avicel^{®} PH | 102-15 |
| HPMC -5- | 4.8 |
| Na croscarmellose | 13 |
| Anhydr. coll. silica -Aerosil^{®} | 200-0.7 |
| Polysorbate 80 | 0.5 |
| Mg stearate | 1 |
| Yellow iron oxide | 1 |
| Red iron oxide | 1 |

The preparation is analogous to that reported above for Example 7.

### EXAMPLE 12

| Preparation of Glibencamide + Metformin (2.5 + 500 mg) bi-layered tablets | |
|---|---|
| Composition of the Metformin layer for each tablet, in mg | |
| Metformin HCl | 500 |
| Macrogol 4000 | 24.7 |
| Anhydrous colloidal silica | 8.2 |
| Polyvinylpyrrolidone | 20 |
| Magnesium Stearate | 5.9 |

| Composition of the Glibenclamide layer for each tablet, in mg: | |
|---|---|
| Glibenclamide | 2.5 |
| Lactose monohydrate | 89 |
| Sodium lauryl sulphate | 0.750 |
| Microcrystalline cellulose | 150 |
| Sodium croscarmellose | 5.500 |
| Anhydrous colloidal silica | 0.750 |
| Magnesium Stearate | 0.750 |
| Yellow iron oxide E 172 | 0.750 |

The preparation procedure is as described in Example 7.

The tablets can be coated with substances well known in the pharmaceutical field.

### EXAMPLE 13

| Preparation of GlibenclamidXe + Metformin (5 + 500 mg) bi-layered tablets | |
|---|---|
| Composition of the Metformin layer for each tablet in mg | |
| Metformin HCl | 500 |
| Macrogol 4000 | 24.7 |
| Anhydrous colloidal silica | 8.2 |
| Polyvinylpyrrolidone | 20 |
| Magnesium Stearate | 5.9 |

| Composition of the glibenclamide layer for each tablet in mg | |
|---|---|
| Glibenclamide | 5 |
| Lactose monohydrate | 86.75 |
| Sodium lauryl sulphate | 0.750 |
| Microcrystalline cellulose | 150 |
| Sodium croscarmellose | 5.500 |
| Anhydrous colloidal silica | 0.750 |
| Magnesium Stearate | 0.750 |
| Yellow iron oxide E 172 | 0.125 |
| Red iron oxide E 172 | 0.375 |

The preparation procedure is as described in Example 7.

The tablets can be coated with substances which are well known in the pharmaceutical field.

### EXAMPLE 14

| Preparation of Glibenclamide + Metformin (2.5 + 850 mg) bi-layered tablets | |
|---|---|
| Composition of the Metformin layer for each tablet in mg | |
| Metformin HCl | 850 |
| Macrogol 4000 | 42 |
| Anhydrous colloidal silica | 14 |
| Polyvinylpyrrolidone | 34 |
| Magnesium stearate | 10 |

| Composition of the glibenclamide layer for each tablet in mg | |
|---|---|
| Glibenclamide | 2.5 |
| Lactose monohydrate | 89 |
| Sodium lauryl sulphate | 0.750 |
| Microcristalline cellulose | 150 |
| Sodium croscarmellose | 5.500 |
| Anhydrous colloidal silica | 0.750 |
| Magnesium Stearate | 0.750 |
| Yellow iron oxide E | 0.750 |

The method of preparation is as described in Example 7.

The tablets can be coated with substances which are well known in the field of pharmaceuticals.

### EXAMPLE 15:

| Preparation of Glibenclamide + Metformin (5 + 850 mg) bi-layered tablets | |
|---|---|
| Composition of the Metformin layer for each tablet in mg | |
| Metformin HCl | 850 |
| Macrogol 4000 | 42 |
| Anhydrous colloidal silica | 14 |
| Polyvinylpyrrolidone | 34 |
| Magnesium stearate | 10 |

| Composition of the glibenclamide layer for each tablet in mg | |
|---|---|
| Glibenclamide | 5 |
| Lactose monohydrate | 86.75 |
| Sodium lauryl sulphate | 0.750 |
| Microcrystalline cellulose | 150 |
| Sodium croscarmellose | 5.500 |
| Anhydrous colloidal silica | 0.750 |
| Magnesium Stearate | 0.750 |
| Yellow iron oxide E 172 | 0.125 |
| Red iron oxide E 172 | 0.375 |

The method of preparation is as described in Example 7.

The tablets can be coated with substances which are well known in the pharmaceutical field.

### DISSOLUTION TEST

The studies on the glibenclamide bioavailability in the various formulations can be uselessly complex, for which for the study of the various formulations the dissolution test has been considered assuming as a first approximation that a greater dissolution in a given defined time corresponds to a greater bioavailablity (J. Pharm. Pharmacol., 2000, 52, 831-838; Drug Development and industrial Pharmacy, 1993, 19 (20), 2713-2741).

The tablet under test is added to an aqueous solution (900 ml) containing 0.05 M phosphate buffer at pH 7.4 at 37°C and dissolved using a paddle at 35 rpm. Unless otherwise specified, a sample of the solution is taken following 45 minutes and the glibenclamide dosaged by HPLC (internal method). The dissolution test is repeated at least 6 times on tablets of the same batch and the mean value is considered.

Experiment 1) Dissolution of commercial compounds
a)
   - Glibenclamide 5 mg37% (DAONIL^{®})
   - Glibenclamide 5 mg + Metformin 500 mg 42% (DAONIL^{®} + GLUCOPHAGE^{®})
   - Glibenclamide 5mg + Metformin 500 mg 39% (EUGLUCON^{®} + GLUCOPHAGE^{®})

   As can be seen from the results reported above, the dissolution of commercial Glibenclamide tablets (Daonil^{®} or Euglucon^{®}) in the presence of a Metformin 500 mg commercial tablet (Glucophage^{®}), does not seem to be different from the dissolution of Glibenclamide tablets alone.
b)
   - Glibenclamide 5 mg58%
      (GLIBORAL^{®})
   - Glibenclamide 5 mg + Metformin 500 mg 52%
      (GLIBORAL^{®} + SIOFOR^{®})
   - Glib+Met (5+500 mg) mixture 26%
      (GLIBOMET^{®})

The dissolution of commercial glibenclamide tablets (Gliboral^{®}) alone or in the presence of a metformin 500 mg commercial tablet (Siofor^{®}) is comparable, whilst the dissolution of glibenclamide in mixture with metformin in a single commercial tablet (Glibomet) is heavily reduced. The commercial products have been selected on the basis of their similarity in composition and the type of excipients used so as to minimise their influence.

### Experiment 2)

- Glib. 5 mg (Example 1) 88%
- Glib. 5 mg + Met. 400 mg (Examples 1 + 2) 92%
- Glib + Met 5 + 400 mg (Example 3) 15%
- Glib + Met 5 + 400 mg (Example 10) 89%

The tablets have the compositions described in the Examples specifically indicated. The excipients which constitute the glibenclamide tablet (Example 1) are qualitatively and quantitatively identical to these which constitute the glibenclamide layer in the bi-layered tablet (Example 10). Analogously, the excipients which constitute the metformin tablet (Example 2) are qualitatively and quantitatively identical to these which constitute the metformin layer in the bi-layered tablet. The excipients in the glibenclamide/metformin mixture tablet (Example 3) are quali/quantitatively equal to the sum of the excipients of the metformin and glibenclamide tablets (Examples 1 and 2). In this manner it was attempted to minimise the effect of the excipients themselves on the dissolution of one tablet or the other.

Glibenclamide, when mixed with metformin, has a notable reduction in the percentage dissolution with respect to when the two compounds are maintained in separate compositions (as already observed for the commercial tablets in experiment 1b). Surprisingly the use of a bi-layered tablet eliminates the problem and the glibenclamide dissolves in comparable percentages with respect to the two separate products.

### Experiment 3)

Composition 5' 15' 45%
- Glib 5 + Met 500 (Example 4)) 54% 65% 66%
- Glib + Met 5+500 (Example 5) 11 % 44% 64%
- Glib + Met 5 + 500 (Example 6) 27% 53% 53%
- Glib + Met 5+500 (Example 11) 52% 86% 103%

In experiment 3) the dissolution values are measured at three different times, i.e. at 5, 15 and 45 minutes. The formulations used in the present experiment, analogously to the preceding experiment 2, have excipient contents comparable so as to eliminate their effects within the specific group examined.

One can effectively note that the excipients can play an important role in dissolution by comparing the dissolution obtained with the mixture of Examples 5 and 6 in comparison with that of 3 or with the commercial examples. With the use of appropriate excipients it is possible to obtain also for mixtures of Glib + Met, dissolution values comparable to these of Glib + Met given separately up to final times of 45'. An analysis can attempt as a function of time, to show however that at short times (following 5') the dissolution of the mixtures is still much lower than these of the separate tablets (11% and 27% as opposed to 54%), but this appears evident also following 15'(44% and 53% as opposed to 65%). These data might not allow an equal bioavailability profile in vivo and therefore different behaviour and pharmacodynamics. One such importance derives also from the method of preparation of the mixtures, in fact at short times, the glibenclamide dissolves more rapidly when the granulation is performed on the already prepared mixture (Example 6) rather than when the two active ingredients are granulated separately and then mixed (Example 5).

However the superiority of the corresponding bi-layered tablets appears very clear (Example 11), the dissolution percentage of which is practically comparable at 5 minutes to that of the two separate tablets, and is always greater in all the other cases.

### Experiment 4) Bi-layered tablets

- Example 7 66%
- Example 8 86%
- Example 9 53%
- Example 10 89%
- Example 11 103%

The dissolution of the glibenclamide in the bi-layered tablets appears always very good, always comparable or superior to these obtained with separate tablets.

## Claims

1. Pharmaceutical formulations for the oral administration in tablet form, comprising glibenclamide and metformin, or pharmaceutically acceptable salts thereof, as active ingredients, **characterised by** the fact that said active ingredients are comprised in layers separated from each other and the amount of glibenclamide in each tablet ranges between 1 and 20 mg and the amount of metformin in each tablet ranges between 200 and 1000 mg.

2. The pharmaceutical formulations according to claim 1, wherein the amount of glibenclamide in each tablet ranges between 2.5 and 5 mg and the amount of metformin in each tablet ranges between 500 and 850 mg.

3. The pharmaceutical formulations according to claims 1 and 2, wherein said tablets are selected from multi-layered tablets and coated tablets wherein the glibenclamide is always located in at least one external layer of the multi-layered tablets or on the coating of the coated tablets.

4. The pharmaceutical formulations according to claim 3, wherein said multi-layered tablets are selected from:
a) bi-layered tablets wherein the glibenclamide is compressed into a relatively thin layer over a tablet of metformin, and
b) tri-layered tablets wherein the glibenclamide is compressed into two layers placed one above and the other below a central layer of metformin.

5. The pharmaceutical formulations according to claim 4, wherein said multi-layered tablets are bi-layered tablets wherein the glibenclamide is compressed into a relatively thin layer over a metformin tablet.

6. The pharmaceutical formulations according to claims 3, wherein said coated tablets are selected from:
a') metformin tablets uniformly coated by a thin layer comprising glibenclamide which is compressed all around it; and
b') metformin tablets externally painted with a solution of glibenclamide which is layered onto it.

7. The pharmaceutical formulations according to the claims from 1 to 6, comprising in addition pharmaceutically acceptable excipients.

8. The pharmaceutical formulations according to claim 7, wherein the part of the formulation comprising glibenclamide comprises at least one surfactant or a hydrosoluble dispersing agent as excipients.

9. The pharmaceutical formulations according to claim 8, wherein the part of the formulation comprising glibenclamide comprises a surfactant at a concentration of from 0.1 to 10 mg as excipient.

10. The pharmaceutical formulations according to claim 9, wherein the concentration of said surfactant is comprised of between 0.5 to 2 mg.

11. The pharmaceutical formulations according to claim 8, wherein said surfactant is selected from the alkaline or alkaline earth salts of lauryl sulphate and Polysorbate 80.

12. The pharmaceutical formulations according to claim 11, wherein said surfactant is sodium lauryl sulphate.

13. The pharmaceutical formulations according to claim 8, wherein the part of the formulation comprising glibenclamide comprises a hydrosoluble dispersing agent at a concentration of between 20 to 100 mg as excipient.

14. The pharmaceutical formulations according to claim 13, wherein the concentration of said hydrosoluble dispersing agent is comprised of between 45 and 55 mg.

15. The pharmaceutical formulations according to claim 8, wherein said hydrosoluble dispersing agent is selected from the polyethylene glycols.

16. The pharmaceutical formulations according to claim 15, wherein said hydrosoluble dispersing agent is Macrogol 6000.

17. The pharmaceutical formulations according to claims 1 and 2, which are:
- Glib + Met (5+500mg)
Metformin layer : Metformin HCl, 500; Macrogol 4000, 24.7; Polivinilpirrolidone, 20; Anhydrous colloidal silica, 5;
Glibenclamide layer : Glibenclamide, 5; Lactose monohydrate, 50.75; Na lauryl sulphate, 0.75 ; Microcrystalline cellulose, 100; Sodium croscarmellose 5.5; Anhydrous colloidal silica 0.5; Magnesium Stearate 3.4; Yellow iron oxide E 172, 1; Red iron oxide E 172,1;
- Glib + Met (5+500mg)
Metformin layer: Metformin HCl 500; Macrogol 4000, 24.7; Anhydr. coll. silica - Aerosil 200, 5;Polyvinylpyrrolidone K 25, 20; Mg stearate, 5.9
Glibenclamide layer: Glibenclamide, 5;Macrogol 6000, 50;Lactose mon. S.D., 34; Microgr. Cell.-Avicel PH 102, 70; Na croscarmellose, 5; Anhydr. coll. silica -Aerosil 200, 0.5; Mg stearate, 0.5
- Glib + Met (5+500 mg)
Metformin layer :Metformin HCl 500; Macrogol 4000 247; Anhydr. coll. silica -Aerosil 200, 5; Polyvinylpyrrolidone K 25, 20;Mg stearate 5
Glibenclamide layer: Glibenclamide 5; Microgr. Cell.-Avicel PH 102, 100; Na croscarmellose, 5.25; Anhydr. coll. silica -Aerosil 200, 0.5; Na lauryl sulphate 0.5; Mg stearate 0.5; Yellow iron oxide, 1; Red iron oxide 0.7
- Glib + Met (5+400mg)
Metformin layer: Metformin HCl, 400; Microcrystalline cellulose 65; Corn starch 60; Anhydrous colloidal silicate 20; Gelatine 40; Glycerine 17.5; Talc 17.5; Magnesium stearate 7.5; Cellulose acetate phthalate 2; Diethylphthalate 0.5;
Glibenclamide layer: Glibenciamide, 5; Pregelatinised corn starch 58.3; Hydrated colloidal silica 0.5; Lactose monohydrate 96.2; Na lauryl sulphate 1; Talc 0.5; Magnesium stearate 0.5;
- Glib + Met (5+500mg)
Metformin layer : Metformin HCl 500; Macrogol 4000, 24.7; anhydr. coll. silica - Aerosil 200, 5; Polyvinylpyrrolidone K 25, 20; Mg stearate, 5.9;
Glibenclamide layer: Glibenclamide 5; Lactose mon.-granul. 128; Corn starch 40; Microgr. Cell.-Avicel PH 102, 15; HPMC -5, 4.8; Na croscarmellose, 13; Anhydr. coll. silica -Aerosil 200, 0.7; Polysorbate 80, 0.5; Mg stearate 1; Yellow iron oxide 1; Red iron oxide 1;
- Glib+ Met (2.5 + 500 mg)
Metformin layer : Metformin HCl 500; Macrogol 4000, 24.7; Anhydrous colloidal silica 8.2; Polyvinylpyrrolidone 20; Magnesium Stearate 5.9;
Glibenclamide layer: Glibenclamide 2.5 ; Lactose monohydrate 89; Sodium lauryl sulphate 0.75; Microcrystalline cellulose 150; Sodium croscarmellose 5.5; Anhydrous colloidal silica 0.75; Magnesium Stearate 0.75; Yellow iron oxide E 172 0.75;
- Glib+ Met (5 + 500 mg)
metformin layer : Metformin HCl 500;Macrogol 4.000, 24.7; Anhydrous colloidal silica 8.2; Polyvinylpyrrolidone 20; Magnesium Stearate 5.9;
glibenclamide layer: Glibenclamide 5; Lactose monohydrate 86.75; Sodium lauryl sulphate 0.750; Microcrystalline cellulose 150; Sodium croscarmellose 5.5; Anhydrous colloidal silica 0.75; Magnesium Stearate 0,75; Yellow iron oxide E 172, 0.125; Red iron oxide E 172, 0.375;
- Glib+ Met (2.5 + 850 mg)
metformin layer: Metformin HCl 850; Macrogol 4000, 42; Anhydrous colloidal silica 14; Polyvinylpyrrolidone 34; Magnesium stearate 10;
glibenclamide layer : Glibenclamide 2.5; Lactose monohydrate 89; Sodium lauryl sulphate 0.75; Microcristalline cellulose 150; Sodium croscarmellose 5.5; Anhydrous colloidal silica 0.750; Magnesium Stearate 0.750; Yellow iron oxide E 172, 0.750
- Glib + Met (5 + 850 mg)
metformin layer : Metformin HCl 850; Macrogol 4000, 42; Anhydrous colloidal silica 14; Polyvinylpyrrolidone 34; Magnesium Stearate 10;
glibenclamide layer: Glibenclamide 5; lactose monohydrate, 86.75; Sodium lauryl sulphate 0.75; Microcrystalline cellulose 150; Sodium croscarmellose 5.5; Anhydrous colloidal silica 0.75; Magnesium Stearate 0.75; Yellow iron oxide E 172, 0.125; Red iron oxide E 172, 0.375;

18. Use of glibenclamide and metformin, or pharmaceutically acceptable salts thereof, for the preparation of pharmaceutical compositions as described in the claims from 1 to 17 for the treatment of type II diabetes mellitus.

## Patentansprüche

1. Pharmazeutische Formulierungen zur oralen Verabreichung in Tablettenform enthaltend Glibenclamid und Metformin oder pharmazeutisch akzeptable Salze hiervon als aktive Bestandteile, **dadurch gekennzeichnet, dass** die aktiven Bestandteile in Schichten, welche voneinander getrennt sind, enthalten sind und die Menge an Glibenclamid in jeder Tablette in einem Bereich zwischen 1 und 20 mg sowie die Menge an Metformin in jeder Tablette in einem Bereich zwischen 200 und 1000 mg liegt.

2. Pharmazeutische Formulierungen nach Anspruch 1, wobei die Menge an Glibenclamid in jeder Tablette in einem Bereich zwischen 2,5 und 5 mg und die Menge an Metformin in jeder Tablette in einem Bereich zwischen 500 und 850 mg liegt.

3. Pharmazeutische Formulierungen nach Anspruch 1 und 2, wobei die Tabletten aus mehrschichtigen Tabletten und aus beschichteten Tabletten ausgewählt sind, wobei das Glibenclamid immer in wenigstens einer äußeren Schicht der mehrschichtigen Tabletten oder auf der Beschichtung der beschichteten Tabletten lokalisiert ist.

4. Pharmazeutische Formulierungen nach Anspruch 3, wobei die mehrschichtigen Tabletten ausgewählt sind aus:
a) zweischichtigen Tabletten, in denen das Glibenclamid zu einer relativ dünnen Schicht über einer Tablette aus Metformin komprimiert ist, und
b) dreischichtigen Tabletten, in denen das Glibenclamid zu zwei Schichten komprimiert ist, von denen eine oberhalb und von denen die andere unterhalb einer zentralen Schicht aus Metformin platziert ist.

5. Pharmazeutische Formulierungen nach Anspruch 4, wobei die mehrschichtigen Tabletten zweischichtige Tabletten sind, in denen das Glibenclamid zu einer relativ dünnen Schicht über einer Metformintablette komprimiert ist.

6. Pharmazeutische Formulierungen nach Anspruch 3, wobei die beschichteten Tabletten ausgewählt sind aus:
a') Metformintabletten, welche mit einer dünnen Schicht enthaltend Glibenclamid, welches überall komprimiert ist, einheitlich beschichtet sind, und
b') Metformintabletten, welche äußerlich mit einer Lösung von Glibenclamid überstrichen sind, welche auf diese geschichtet ist.

7. Pharmazeutische Formulierungen nach den Ansprüchen 1 bis 6 enthaltend des Weiteren pharmazeutisch akzeptable Hilfsstoffe.

8. Pharmazeutische Formulierungen nach Anspruch 7, wobei der Glibenclamid enthaltende Teil der Formulierung als Hilfsstoffe wenigstens ein Tensid oder ein wasserlösliches Dispergiermittel enthält.

9. Pharmazeutische Formulierungen nach Anspruch 8, wobei der Glibenclamid enthaltende Teil der Formulierung als Hilfsmittel ein Tensid in einer Konzentration zwischen 0,1 und 10 mg enthält.

10. Pharmazeutische Formulierungen nach Anspruch 9, wobei die Konzentration des Tensids zwischen 0,5 und 2 mg beträgt.

11. Pharmazeutische Formulierungen nach Anspruch 8, wobei das Tensid aus Alkali- oder Erdalkalisalzen von Laurylsulfat und Polysorbat 80 ausgewählt ist.

12. Pharmazeutische Formulierungen nach Anspruch 11, wobei das Tensid Natriumlaurylsulfat ist.

13. Pharmazeutische Formulierungen nach Anspruch 8, wobei der Glibenclamid enthaltende Teil der Formulierung als Hilfsmittel ein wasserlösliches Dispergiermittel in einer Konzentration zwischen 20 und 100 mg enthält.

14. Pharmazeutische Formulierungen nach Anspruch 13, wobei die Konzentration des wasserlöslichen Dispergiermittels zwischen 45 und 55 mg beträgt.

15. Pharmazeutische Formulierungen nach Anspruch 8, wobei das wasserlösliche Dispergiermittel aus Polyethylenglykolen ausgewählt ist.

16. Pharmazeutische Formulierungen nach Anspruch 15, wobei das wasserlösliche Dispergiermittel Macrogol 6000 ist.

17. Pharmazeutische Formulierungen nach Anspruch 1 und 2, welche sind:
- Glib + Met (5 + 500 mg)
Metforminschicht: Metformin HCl, 500; Macrogol 4000, 24,7; Polyvinylpyrrolidon, 20; wasserfreie kolloidale Kieselsäure, 5
Glibenclamidschicht: Glibenclamid, 5; Lactosemonohydrat, 50,75; Na-Laurylsulfat, 0,75; mikrokristalline Cellulose 100; Natriumcroscarmellose 5,5; wasserfreie kolloidale Kieselsäure 0,5; Magnesiumstearat 3,4; gelbes Eisenoxid E 172, 1; rotes Eisenoxid E 172, 1
- Glib + Met (5 + 500 mg)
Metforminschicht: Metformin HCl, 500; Macrogol 4000, 24,7; wasserfreie kolloidale Kieselsäure-Aerosil 200, 5; Polyvinylpyrrolidon K 25, 20; Mg-Stearat, 5,9
Glibenclamidschicht: Glibenclamid, 5; Macrogol 6000, 50; Lactosemonohydrat S.D., 34; mikrokristalline Cellulose-Avicel PH 102, 70; Na-Croscarmellose 5; wasserfreie kolloidale Kieselsäure-Aerosil 200, 0,5; Mg-Stearat, 0,5
- Glib + Met (5 + 500 mg)
Metforminschicht: Metformin HCl 500; Macrogol 4000, 247; wasserfreie kolloidale Kieselsäure-Aerosil 200, 5; Polyvinylpyrrolidon K 25, 20; Mg-Stearat, 5
Glibenclamidschicht: Glibenclamid 5; mikrokristalline Cellulose-Avicel PH 102, 100; Na-Croscarmellose, 5,25; wasserfreie kolloidale Kieselsäure-Aerosil 200, 0,5; Na-Laurylsulfat 0,5; Mg-Stearat 0,5; gelbes Eisenoxid, 1; rotes Eisenoxid 0,7
- Glib + Met (5 + 400 mg)
Metforminschicht: Metformin HCl, 400; mikrokristalline Cellulose 65; Maisstärke 60; wasserfreies kolloidales Silikat 20; Gelatine 40; Glycerin 17,5; Talk 17,5; Magnesiumstearat 7,5; Celluloseacetatphthalat 2; Diethylphthalat 0,5
Glibenclamidschicht: Glibenclamid, 5; vorgelatinierte Maisstärke 58,3; hydratisierte kolloidale Kieselsäure 0,5; Lactosemonohydrat 96,2; Na-Laurylsulfat 1; Talk 0,5; Magnesiumstearat 0,5
- Glib + Met (5 + 500 mg)
Metforminschicht: Metformin HCl 500; Macrogol 4000, 24,7; wasserfreie kolloidale Kieselsäure-Aerosil 200, 5; Polyvinylpyrrolidon K 25, 20; Mg-Stearat, 5,9
Glibenclamidschicht: Glibenclamid 5; Lactosemonohydrat granul. 128; Maisstärke 40; mikrokristalline Cellulose-Avicel PH 102, 15; HPMC -5, 4,8; Na-Croscarmellose, 13; wasserfreie kolloidale Kieselsäure-Aerosil 200, 0,7; Polysorbat 80, 0,5; Mg-Stearat 1; gelbes Eisenoxid 1; rotes Eisenoxid 1
- Glib + Met (2,5 + 500 mg)
Metforminschicht: Metformin HCl 500; Macrogol 4000, 24,7; wasserfreie kolloidale Kieselsäure 8,2; Polyvinylpyrrolidon 20; Magnesiumstearat 5,9
Glibenclamidschicht: Glibenclamid 2,5; Lactosemonohydrat 89; Natriumlaurylsulfat 0,75; mikrokristalline Cellulose 150; Natriumcroscarmellose 5,5; wasserfreie kolloidale Kieselsäure 0,75; Magnesiumstearat 0,75; gelbes Eisenoxid E 172 0,75
- Glib + Met (5 + 500mg)
Metforminschicht: Metformin HCl 500; Macrogol 4000, 24,7; wasserfreie kolloidale Kieselsäure 8,2; Polyvinylpyrrolidon 20; Magnesiumstearat 5,9
Glibenclamidschicht: Glibenclamid 5; Lactosemonohydrat 86,75; Natriumlaurylsulfat 0,750; mikrokristalline Cellulose 150; Natriumcroscarmellose 5,5; wasserfreie kolloidale Kieselsäure 0,75; Magnesiumstearat 0,75; gelbes Eisenoxid E 172, 0,125; rotes Eisenoxid E 172, 0,375
- Glib + Met (2,5 + 850mg)
Metforminschicht: Metformin HCl 850; Macrogol 4000, 42; wasserfreie kolloidale Kieselsäure 14; Polyvinylpyrrolidon 34; Magnesiumstearat 10
Glibenclamidschicht: Glibenclamid 2,5; Lactosemonohydrat 89; Natriumlaurylsulfat 0,75; mikrokristalline Cellulose 150; Natriumcroscarmellose 5,5; wasserfreie kolloidale Kieselsäure 0,750; Magnesiumstearat 0,750; gelbes Eisenoxid E 172, 0,750
- Glib + Met (5 + 850 mg)
Metforminschicht: Metformin HCl 850; Macrogol 4000, 42; wasserfreie kolloidale Kieselsäure 14; Polyvinylpyrrolidon 34; Magnesiumstearat 10
Glibenclamidschicht: Glibenclamid 5; Lactosemonohydrat 86,75; Natriumlaurylsulfat 0,75; mikrokristalline Cellulose 150; Natriumcroscarmellose 5,5; wasserfreie kolloidale Kieselsäure 0,75; Magnesiumstearat 0,75; gelbes Eisenoxid E 172, 0,125; rotes Eisenoxid E 172, 0,375

18. Verwendung von Glibenclamid und Metformin oder pharmazeutisch akzeptablen Salzen hiervon zur Herstellung von pharmazeutischen Zusammensetzungen nach einem der Ansprüche 1 bis 17 zur Behandlung von Diabetes mellitus Typ II.

## Revendications

1. Formulations pharmaceutiques pour l'administration orale sous forme de comprimés, comprenant du glibenclamide et de la metformine, ou des sels pharmaceutiquement acceptables de ceux-ci, **caractérisées par le fait que** lesdits principes actifs sont compris dans des couches séparées les unes des autres et que la quantité de glibenclamide dans chaque comprimé se situe dans la plage entre 1 et 20 mg et que la quantité de metformine dans chaque comprimé se situe dans la plage entre 200 et 1000 mg.

2. Formulations pharmaceutiques selon la revendication 1, dans lesquelles la quantité de glibenclamide dans chaque comprimé se situe dans la plage entre 2,5 et 5 mg et la quantité de metformine dans chaque comprimé se situe dans la plage entre 500 et 850 mg.

3. Formulations pharmaceutiques selon les revendications 1 et 2, où lesdits comprimés sont choisis parmi des comprimés multicouches et des comprimés enrobés dans lesquels le glibenclamide est toujours situé dans au moins une couche externe des comprimés multicouches ou sur l'enrobage des comprimés enrobés.

4. Formulations pharmaceutiques selon la revendication 3, où lesdits comprimés multicouches sont choisis parmi :
a) des comprimés bicouches dans lesquels le glibenclamide est compressé en une couche relativement fine sur un comprimé de metformine, et
b) des comprimés tricouches dans lesquels le glibenclamide est compressé en deux couches placées l'une au-dessus de l'autre au-dessous d'une couche centrale de metformine.

5. Formulations pharmaceutiques selon la revendication 4, où lesdits comprimés multicouches sont des comprimés bicouches dans lesquels le glibenclamide est compressé en une couche relativement fine sur un comprimé de metformine.

6. Formulations pharmaceutiques selon la revendication 3, où lesdits comprimés enrobés sont choisis parmi :
a') des comprimés de metformine uniformément enrobés d'une couche fine comprenant du glibenclamide qui est compressée tout autour d'eux ; et
b') des comprimés de metformine peints en externe avec une solution de glibenclamide qui est déposée en couche sur eux.

7. Formulations pharmaceutiques selon les revendications 1 à 6, comprenant en plus des excipients pharmaceutiquement acceptables.

8. Formulations pharmaceutiques selon la revendication 7, dans lesquelles la partie de la formulation comprenant le glibenclamide comprend au moins un surfactant ou un agent de dispersion hydrosoluble comme excipients.

9. Formulations pharmaceutiques selon la revendication 8, dans lesquelles la partie de la formulation comprenant le glibenclamide comprend un surfactant à une concentration de 0,1 à 10 mg comme excipient.

10. Formulations pharmaceutiques selon la revendication 9, dans lesquelles la concentration dudit surfactant est comprise entre 0,5 et 2 mg.

11. Formulations pharmaceutiques selon la revendication 8, dans lesquelles ledit surfactant est choisi parmi des sels de métaux alcalins ou alcalino-terreux de laurylsulfate et le Polysorbate 80.

12. Formulations pharmaceutiques selon la revendication 11, dans lesquelles ledit surfactant est le laurylsulfate de sodium.

13. Formulations pharmaceutiques selon la revendication 8, dans lesquelles la partie de la formulation comprenant le glibenclamide comprend un agent de dispersion hydrosoluble à une concentration entre 20 et 100 mg comme excipient.

14. Formulations pharmaceutiques selon la revendication 13, dans lesquelles la concentration dudit agent de dispersion hydrosoluble est comprise entre 45 et 55 mg.

15. Formulations pharmaceutiques selon la revendication 8, dans lesquelles ledit agent de dispersion hydrosoluble est choisi parmi les polyéthylène glycols.

16. Formulations pharmaceutiques selon la revendication 15, dans lesquelles ledit agent de dispersion hydrosoluble est le Macrogol 6000.

17. Formulations pharmaceutiques selon les revendications 1 et 2, qui sont:
- Glib + Met (5 + 500 mg)
couche de metformine: metformine HCl, 500; Macrogol 4000, 24,7 ; polyvinylpyrrolidone, 20; silice colloïdale anhydre, 5 ;
couche de glibenclamide : glibenclamide, 5 ; lactose monohydraté, 50,75 ; laurylsulfate de Na, 0,75 ; cellulose microcristalline, 100 ; croscarmellose sodique, 5,5 ; silice colloïdale anhydre, 0,5 ; stéarate de magnésium, 3,4 ; oxyde de fer jaune E 172, 1 ; oxyde de fer rouge E 172, 1 ;
- Glib + Met (5 + 500 mg)
couche de metformine: metformine HCl, 500 ; Macrogol 4000, 24,7 ; silice colloïdale anhydre - Aerosil 200, 5 ; polyvinylpyrrolidone K 25, 20 ; stéarate de Mg, 5,9 ;
couche de glibenclamide : glibenclamide, 5; Macrogol 6000, 50 ; lactose monohydraté S.D., 34 ; cellulose microcristalline - Avicel PH 102, 70 ; croscarmellose sodique, 5 ; silice colloïdale anhydre - Aerosil 200, 0,5 ; stéarate de Mg, 0,5 ;
- Glib + Met (5 + 500 mg)
couche de metformine: metformine HCl, 500; Macrogol 4000, 247 ; silice colloïdale anhydre - Aerosil 200, 5 ; polyvinylpyrrolidone K 25, 20 ; stéarate de Mg, 5 ;
couche de glibenclamide : glibenclamide, 5 ; cellulose microcristalline - Avicel PH 102, 100 ; croscarmellose sodique, 5,25 ; silice colloïdale anhydre - Aerosil 200, 0,5 ; laurylsulfate de Na, 0,5 ; stéarate de Mg, 0,5 ; oxyde de fer jaune, 1 ; oxyde de fer rouge, 0,7;
- Glib + Met (5 + 400 mg)
couche de metformine : metformine HCl, 400 ; cellulose microcristalline, 65 ; amidon de maïs, 60 ; silicate colloïdal anhydre, 20 ; gélatine, 40 ; glycérine, 17,5 ; talc, 17,5 ; stéarate de magnésium, 7,5 ; acétate phtalate de cellulose, 2 ; phtalate de diéthyle, 0,5 ;
couche de glibenclamide : glibenclamide, 5 ; amidon de maïs prégélatinisé, 58,3 ; silice colloïdale hydratée, 0,5 ; lactose monohydraté, 96,2; laurylsulfate de Na, 1; talc, 0,5 ; stéarate de magnésium, 0,5 ;
- Glib + Met (5 + 500 mg)
couche de metformine : metformine HCl, 500 ; Macrogol 4000, 24,7 ; silice colloïdale anhydre - Aerosil 200, 5 ; polyvinylpyrrolidone K 25, 20 ; stéarate de Mg, 5,9 ;
couche de glibenclamide : glibenclamide, 5; lactose monohydraté - granul., 128 ; amidon de maïs, 40 ; cellulose microcristalline - Avicel PH 102, 15 ; HPMC-5, 4,8 ; croscarmellose sodique, 13; silice colloïdale anhydre - Aerosil 200, 0,7 ; Polysorbate 80, 0,5 ; stéarate de Mg, 1; oxyde de fer jaune, 1 ; oxyde de fer rouge, 1;
- Glib + Met (2,5 + 500 mg)
couche de metformine : metformine HCl, 500 ; Macrogol 4000, 24,7 ; silice colloïdale anhydre, 8,2 ; polyvinylpyrrolidone, 20 ; stéarate de magnésium, 5,9 ;
couche de glibenclamide : glibenclamide, 2,5 ; lactose monohydraté, 89 ; laurylsulfate de sodium, 0,75 ; cellulose microcristalline, 150 ; croscarmellose sodique, 5,5 ; silice colloïdale anhydre, 0,75 ; stéarate de magnésium, 0,75 ; oxyde de fer jaune E 172, 0,75 ;
- Glib + Met (5 + 500 mg)
couche de metformine: metformine HCl, 500 ; Macrogol 4000, 24,7 ; silice colloïdale anhydre, 8,2 ; polyvinylpyrrolidone, 20 ; stéarate de magnésium, 5,9 ;
couche de glibenclamide : glibenclamide, 5 ; lactose monohydraté, 86,75 ; laurylsulfate de sodium, 0,750 ; cellulose microcristalline, 150 ; croscarmellose sodique, 5,5 ; silice colloïdale anhydre, 0,75 ; stéarate de magnésium, 0,75 ; oxyde de fer jaune E 172, 0,125 ; oxyde de fer rouge E 172, 0,375 ;
- Glib + Met (2,5 + 850 mg)
couche de metformine: metformine HCl, 850 ; Macrogol 4000, 42 ; silice colloïdale anhydre, 14 ; polyvinylpyrrolidone, 34 ; stéarate de magnésium, 10 ;
couche de glibenclamide : glibenclamide, 2,5 ; lactose monohydraté, 89 ; laurylsulfate de sodium, 0,75 ; cellulose microcristalline, 150 ; croscarmellose sodique, 5,5 ; silice colloïdale anhydre, 0,750 ; stéarate de magnésium, 0,750 ; oxyde de fer jaune E 172, 0,750 ;
- Glib + Met (5 + 850 mg)
couche de metformine: metformine HCl, 850 ; Macrogol 4000, 42; silice colloïdale anhydre, 14 ; polyvinylpyrrolidone, 34 ; stéarate de magnésium, 10 ;
couche de glibenclamide : glibenclamide, 5 ; lactose monohydraté, 86,75 ; laurylsulfate de sodium, 0,75 ; cellulose microcristalline, 150 ; croscarmellose sodique, 5,5 ; silice colloïdale anhydre, 0,75 ; stéarate de magnésium, 0,75 ; oxyde de fer jaune E 172, 0,125 ; oxyde de fer rouge E 172, 0,375.

18. Utilisation de glibenclamide et de metformine, ou de sels pharmaceutiquement acceptables de ceux-ci, pour la préparation de compositions pharmaceutiques telles que décrites dans les revendications 1 à 17 pour le traitement du diabète sucré de type II.
